# EUROPEAN PATENT APPLICATION

(11) **EP 4 123 304 A1**
(43) Date of publication of application: **25.01.2023**
(21) Application number: 21771308.0
(22) Date of filing: 19.03.2021
(51) Int. Cl.: G01N 33/543, G01N 27/327, B01L 3/00

(54) **NANOWELL DIAGNOSTIC KIT FOR DIAGNOSING CARDIOVASCULAR DISEASE SUBSTANCES IN BLOOD**

(30) Priority: 20.03.2020 US 202062992167 P; 02.04.2020 KR 20200040475
(71) Applicant: Mara Nanotech Korea, Inc., Daejeon 34134 (KR)
(72) Inventor: LEE, Hea Yeon, Busan 48300 (KR)
(74) Representative: Mewburn Ellis LLP
(86) International application number: PCT/KR2021/003444
(87) International publication number: WO 2021/187951

(57) **Abstract**

The present invention relates to a nanowell diagnostic kit for diagnosing a cardiovascular disease substance in blood, including: an electrode on which a plurality of nanowells are formed to accommodate a target substance; and a marker fixed to each of the plurality of nanowells of the electrode and responding to the target substance, wherein the target substance is identified through an electrochemical analysis method by applying a current to the electrode.

## Description

### TECHNICAL FIELD

The present invention relates to a nanowell diagnostic kit for diagnosing a cardiovascular disease substance in blood, and more particularly, to a nanowell diagnostic kit for diagnosing a cardiovascular disease substance in blood by performing an electrochemical analysis method using a diagnostic kit having a nanowell electrode formed therein to quantify a very small amount of a target cardiovascular substance in blood, thereby enabling *in vitro* diagnosis.

### BACKGROUND ART

Recently, research on a biosensor for diagnosing a specific molecule has been actively carried out, but technical development of a biosensor for diagnosing a specific molecule in blood is insufficient.

Since specific molecules in blood have a great interference phenomenon with other molecules in blood, have multiple non-specific bindings, and have an extremely low concentration, there are difficulties to develop a biosensor for diagnosing a specific molecule in blood.

Although the risk of cardiovascular diseases is rapidly increasing due to changes in recently changed diet, habits, and the like, a target substance for measuring a cardiovascular disease is present in a very small amount in blood, and thus measurement of such a target substance is difficult.

The existence of a cardiovascular diagnostic marker, such as troponin, CRP, or the like, for measuring an incidence of disease caused by cardiovascular diseases is known, but special equipment is required in a hospital for the measurement and analysis of the marker.

However, a patient with cardiovascular disease is a high-risk patient with potential risk that needs to be monitored at all times so that a quick and simple measurement is required. Thus, there is a need to develop a cardiovascular diagnostic kit that enables quick and simple measurement.

### DESCRIPTION OF EMBODIMENTS

### TECHNICAL PROBLEM

The present invention is created to solve the above-mentioned problems. More specifically, the present invention relates to a nanowell diagnostic kit for diagnosing a cardiovascular disease substance in blood by performing an electrochemical analysis method using a diagnostic kit having a nanowell electrode formed therein to quantify a very small amount of a target cardiovascular substance in blood, thereby enabling *in vitro* diagnosis.

### SOLUTION TO PROBLEM

The nanowell diagnostic kit for diagnosing a cardiovascular disease substance in blood according to the present invention designed to solve the above-mentioned problems includes: an electrode on which a plurality of nanowells are formed to accommodate a target substance; and a marker fixed to each of the plurality of nanowells of the electrode and responding to the target substance, wherein the target substance may be identified according to an electrochemical analysis method by applying a current to the electrode.

In the nanowell diagnostic kit for diagnosing a cardiovascular disease substance in blood of the present invention designed to solve the above-mentioned problems, the target substance may be a target cardiovascular substance that can identify a cardiovascular disease, and the marker may be a cardiovascular marker that responds to the target cardiovascular substance.

In the nanowell diagnostic kit for diagnosing a cardiovascular disease substance in blood according to the present invention designed to solve the above-mentioned problems, the electrochemical analysis method may be performed in a way that, after the cardiovascular marker reacts with the target cardiovascular substance, a probe binding to the target cardiovascular substance and an electron transfer-activating substance generating a current through an oxidation-reduction reaction while being bound to the probe may be inserted into the plurality of nanowells, and then a current value may be read by applying a designated voltage to the electrode to identify the target cardiovascular disease substance.

In the nanowell diagnostic kit for diagnosing a cardiovascular disease substance in blood according to the present invention designed to solve the above-mentioned problems, the electron transfer-activating substance may consist of any one of ferrocene, methylene blue, and ferrate.

In the nanowell diagnostic kit for diagnosing a cardiovascular disease substance in blood according to the present invention designed to solve the above-mentioned problems, the electrode on which the plurality of nanowells are formed may consists of an 8-channel electrode, wherein different markers are fixed to different channels of the electrode.

In the nanowell diagnostic kit for diagnosing a cardiovascular disease substance in blood according to the present invention designed to solve the above-mentioned problems, the electrode may consist of a working electrode, a counter electrode, and a reference electrode. The working electrode may be in the shape of a square. The counter electrode may be in the shape of a rectangular strip, and may be provided to wrap the working electrode from one side to the other side through the lower side of the working electrode. The reference electrode may be in the shape of a square, and may be provided on one side of the working electrode.

In the nanowell diagnostic kit for diagnosing a cardiovascular disease substance in blood according to the present invention designed to solve the above-mentioned problems, the size of each of the plurality of nanowells may be in a range of about 50 nm to about 500 nm.

In the nanowell diagnostic kit for diagnosing a cardiovascular disease substance in blood according to the present invention designed to solve the above-mentioned problems, particles treated with a fluorescent substance may be inserted into each of the plurality of nanowells.

In the nanowell diagnostic kit for diagnosing a cardiovascular disease substance in blood according to the present invention designed to solve the above-mentioned problems, the size of each the particles may be larger than 1/2 of the diameter of each of the plurality of nanowells, and may be smaller than the diameter of each of the plurality of nanowells.

In the nanowell diagnostic kit for diagnosing a cardiovascular disease substance in blood according to the present invention designed to solve the above-mentioned problems, one of the particles may be inserted into one of the plurality of nanowells to proceed a 1:1 reaction.

### ADVANTAGEOUS EFFECTS OF DISCLOSURE

The present invention relates to a nanowell diagnostic kit for diagnosing a cardiovascular disease substance in blood. An electrochemical analysis method may be performed by using a diagnostic kit including a nanowell electrode formed therein, and in this regard, a very small amount of a target cardiovascular substance in blood may be quantified, thereby enabling *in vitro* diagnosis.

In addition, the present invention may have advantages that use of a probe and an electron transfer-activating substance together with a nanowell electrode to perform an electrochemical analysis method may enable quick and simple diagnosis of a cardiovascular disease substance in blood.

In addition, the present invention may have advantages that, through the 1:1 reaction between the fluorescent substance-treated particles and the plurality of nanowells, an electrochemical analysis method and an optical method may be implemented in a single electrode at the same time.

### BRIEF DESCRIPTION OF DRAWINGS

FIG. 1A is a diagram of an 8-channel electrode according to an embodiment of the present invention, and FIG. 1B is a diagram of an electrode on which a nanowell structure is formed according to an embodiment of the present invention.
FIG. 2 is a diagram illustrating a process of manufacturing an electrode on which a nanowell structure is formed according to an embodiment of the present invention.
FIGS. 3A to 3C are each a diagram showing measurements of a target cardiovascular substance by performing an electrochemical analysis method using a diagnostic kit according to an embodiment of the present invention.
FIG. 4A is a diagram illustrating a label-free electrochemical analysis method, and FIG. 4B is a diagram illustrating an electrochemical analysis method using a probe and an electron transfer-activating substance according to an embodiment of the present invention.
FIG. 5 is a diagram of a working electrode, a counter electrode, and a reference electrode according to an embodiment of the present invention.
FIG. 6A is a diagram showing insertion of fluorescent substance-treated particles into an electrode on which a nanowell structure is not formed, and FIG. 6B is a diagram showing insertion of fluorescent substance-treated particles into a nanowell structure according to an embodiment of the present invention.

### MODE OF DISCLOSURE

The present specification clarifies the scope of the present invention, explains the principles of the present invention to enable one of ordinary skill in the art to practice the present invention, and discloses embodiments. The disclosed embodiments may be implemented in various forms.

Expressions "comprises" or "includes" used in various embodiments of the present invention indicate the presence of corresponding functions, operations, or components disclosed herein, and do not limit one or more additional functions, operations, components, and the like. In addition, in various embodiments of the present disclosure, the terms "comprises" or "includes" are intended to designate that the presence of features, numbers, operations, components, parts, or a combination thereof described in the specification. It should be understood that these terms do not preclude the possibility of presence or addition of one or more other numbers, steps, operations, components, parts, or a combination thereof.

When a component is referred to as being "connected" or "coupled" to another component, it should be construed that the component may be directly connected or coupled to the another component or new other components may be present between the component and the another component. On the other hand, when a component is referred to as being "directly connected" or "directly coupled" to another component, it should be construed that new other components are not present between the component and the another component.

The terms "first, second, and the like" used in the present specification may be used to describe various components, but the components should not be limited by the terms. The terms are only used to distinguish one component from other components.

The present invention relates to a nanowell diagnostic kit for diagnosing a cardiovascular disease substance in blood, and more particularly, to a nanowell diagnostic kit for diagnosing a cardiovascular disease substance in blood by performing an electrochemical analysis method using a diagnostic kit in which a nanowell electrode is formed to quantify a very small amount of a target cardiovascular substance in blood, thereby enabling *in vitro* diagnosis . Hereinafter, preferred embodiments of the present invention will be described in detail with reference to the accompanying drawings.

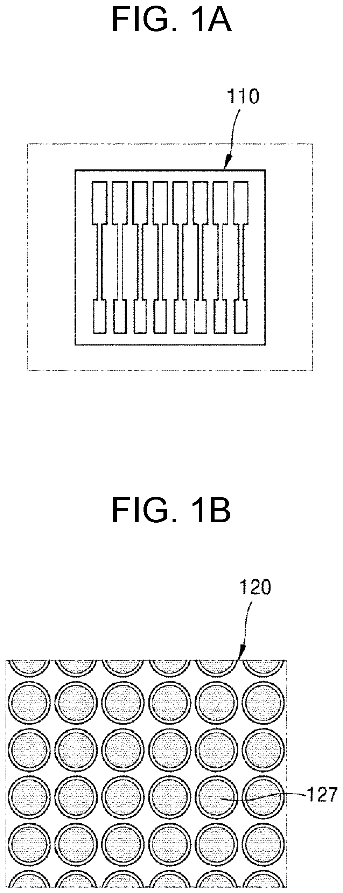

Referring to FIGS. 1A and 1B, a nanowell diagnostic kit for diagnosing a cardiovascular disease substance in blood according to an embodiment of the present invention includes an electrode 120 on which nanowells are formed and a marker. The diagnostic kit according to an embodiment of the present invention may identify a very small amount of a target substance in blood through a nanowell structure 127, and in this regard, the target substance in blood may be identified through an electrochemical analysis method by applying a current to the electrode 120.

Referring to FIG. 1A, in the diagnostic kit according to an embodiment of the present invention, the electrode 120 on which the nanowells are formed may consist of an 8-channel electrode, wherein different markers may be fixed to each channel of the electrode 120. In the diagnostic kit according to an embodiment of the present invention, markers that are different from each other may be fixed to each channel of the electrode 120 to identify various target substances at the same time.

In the diagnostic kit according to an embodiment of the present invention, the target substance may be a target cardiovascular substance 132 that identifies a cardiovascular disease, and the marker may be a cardiovascular marker 131 that responds to the target cardiovascular substance 132. Hereinafter, the diagnostic kit according to an embodiment of the present invention will be described in detail with reference to the target cardiovascular substance 132 and the cardiovascular marker 131.

Referring to FIG. 1B, the electrode 120 on which nanowells are formed according to an embodiment of the present invention refers to an electrode with a nanowell structure 127. The nanowell structure 127 may have a plurality of nanowells consisting of nano-sized grooves, and the size (diameter) of each of the plurality of nanowells may be in a range of about 50 nm to about 500 nm.

The nanowell structure 127 may be formed on the working electrode 141 of the electrode 120, and an oxide film on which the nanowell structure 127 is formed may be provided on the working electrode 141. As the oxide film on which the nanowell structure 127 is formed is provided on the working electrode 141, the resolution may be improved, and accordingly, a very small amount of the target cardiovascular substance 132 may be identified.

The nanowell structure 127 may have a structure in which a plurality of nano-sized grooves are formed in the oxide film (SiO₂) provided on the working electrode 141, and may be prepared by the following method.

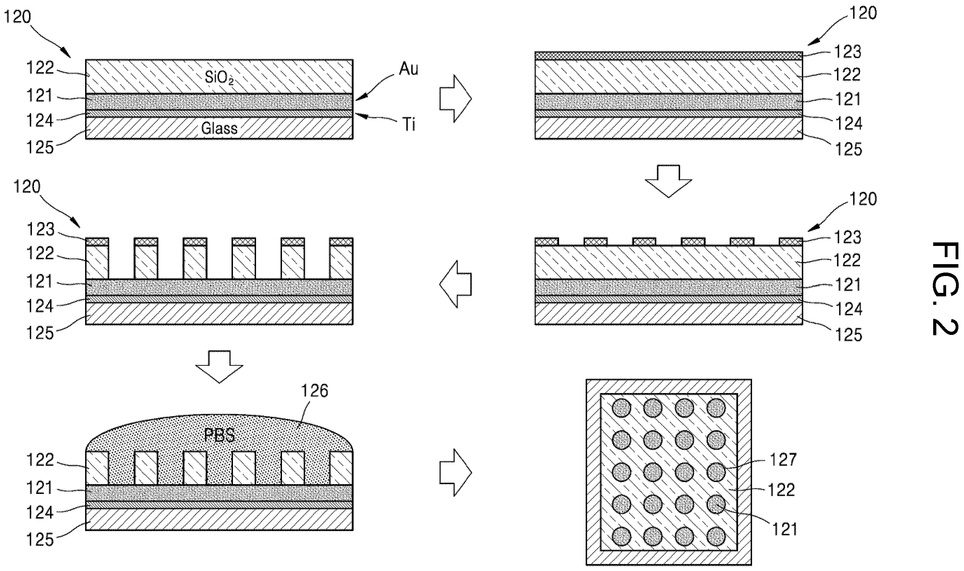

Referring to FIG. 2 , the working electrode 141 on which the nanowell structure 127 is formed may be prepared by a deposit step, a removal step, and an etching step. The deposit step may be a step of forming an oxide layer 122 on a metal layer 121 and spin-coating the oxide layer 122 with a photoresist (PR) 123.

Here, the metal layer 121 may be formed of gold (Au) that is used as the working electrode 120, and the oxide layer may be formed of SiO₂. In addition, a glass 125 may be provided under the metal layer 121, and a sacrificial layer 124 formed of titanium (Ti) may be provided to deposit the metal layer 121 on the glass 125. However, a substance used for the working electrode 141 is not limited to the substance above, and other substances may be used as necessary.

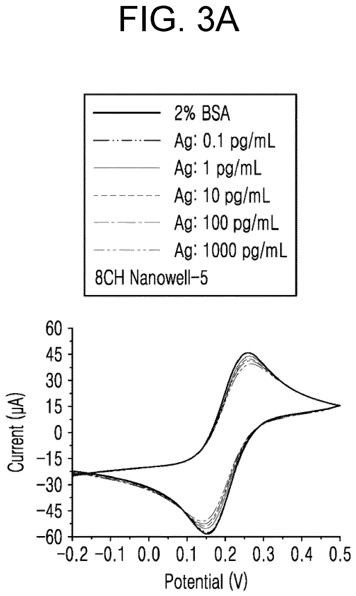

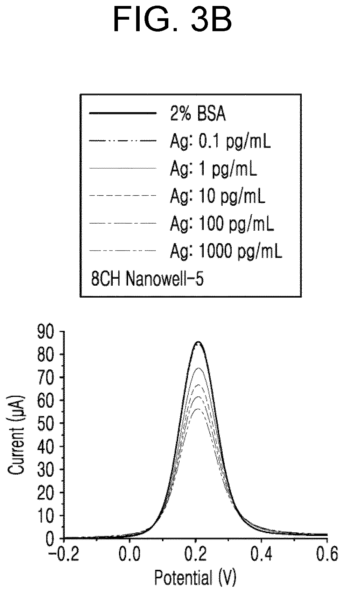

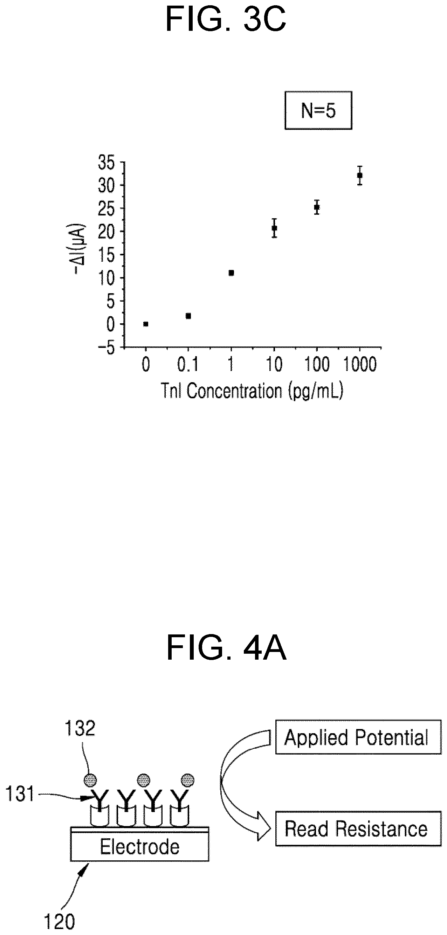

Referring to FIG. 3, the removal step may be a step of removing the photoresist PR 123 in the shape of a nanowell structure by using a photomask. The removal step may be a pre-treatment process for forming the nanowell structure 127 on the oxide layer 122 by performing a development process while leaving only a portion necessary for the photoresist by using a photomask.

Referring to FIG. 3, the etching step may be a step of forming the nanowell structure 127 on the oxide layer 122 by etching the oxide layer 122 through dry etching. Since the photoresist PR 123 is removed to have a nanowell structure from the removal step, the nanowell structure 127 may be formed on the oxide layer 122 when the oxide layer 122 is etched by dry etching in the etching step.

The working electrode 120 that has undergone the etching step may undergo a coating step of forming a coating layer 126 by coating the working electrode 141 while removing the photoresist PR 143, and accordingly, the working electrode 141 on which the nanowell structure 127 is formed may be prepared.

The diagnostic kit according to an embodiment of the present invention may identify a very small amount of a target substance in blood through the nanowell structure 127. By applying a current to the electrode 120, the target substance may be identified by an electrochemical analysis method.

The diagnostic kit according to an embodiment of the present invention may identify a target cardiovascular disease substance by using an electrochemical analysis method to measure changes in current and impedance depending on multiple antigen-antibody reactions or complementary binding to DNA and RNA. In this regard, various electrochemical analysis methods may be used.

FIGS. 3A, 3B, and 3C each show the measurement of a target cardiovascular substance through an electrochemical analysis method. The diagnostic kit according to an embodiment of the present invention may identify a target cardiovascular substance in blood through various electrochemical methods such as cyclic voltammetry, square wave voltammetry (SWQ), and the like.

However, the electrochemical analysis method used in the diagnostic kit according to the embodiment of the present invention may use the following method for quick and simple diagnosis.

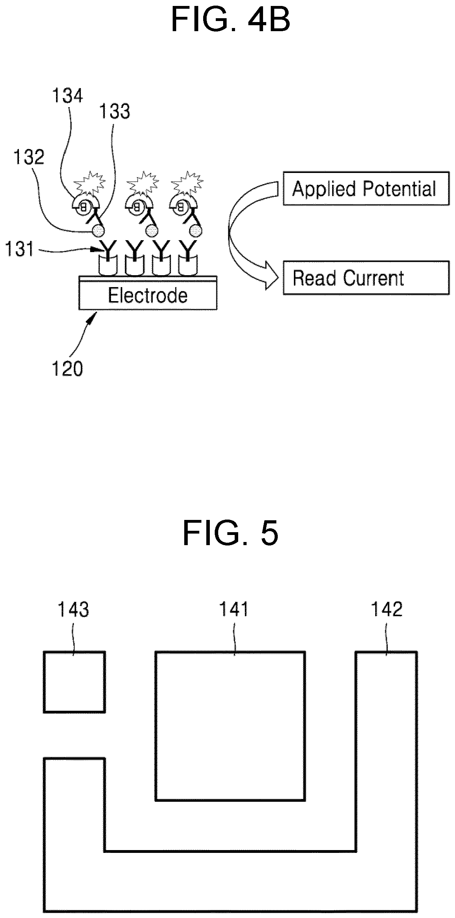

Referring to FIG. 4B, in the electrochemical analysis method according to an embodiment of the present invention, the cardiovascular marker 131 may react to the target cardiovascular substance 132 first. Afterwards, a probe 133 which binds to the target cardiovascular substance 132 and an electron transfer-activating substance 134 which generates a current through an oxidation-reduction reaction while being bound to the probe 133 may be inserted into the nanowells.

Next, by means of reading a current value by applying a designated voltage to the electrode 120, the target cardiovascular substance 132 may be identified. Here, the target cardiovascular substance 132 may be an antigen, and the cardiovascular marker 131 fixed to the electrode 120 may be an antibody responding to the antigen.

The probe 133 may be a secondary antibody that may respond to the antigen, and the electron transfer-activating substance 134 may be a substance that generates a current through an oxidation-reduction reaction with the probe 133. In detail, the electron transfer-activating substance 134 may include ferrocene, methylene blue, ferrate, or the like. However, the electron transfer-activating substance 134 is not limited thereto, and as long as a substance generates a current through an oxidation-reduction reaction with the probe 133, the electron transfer-activating substance 134 may include various substances.

The electrochemical analysis method in the art as shown in FIG. 4A may be a label-free method performed in a way that a marker 131 consisting of an antibody is fixed to an electrode 120, a target substance 132 consisting of an antigen is inserted, and impedance is measured by applying an alternating voltage.

In this way, a sample preparation process may be simple. However, since the impedance needs to be interpreted, the analysis takes a long time due to a complicated way for the analysis, and an expensive apparatus is required to apply an alternating voltage.

However, regarding the electrochemical analysis method according to an embodiment of the present invention, the target substance may be identified by means of reading a current value after applying only a designated voltage to the electrode 120 while using the probe 133 and the electron transfer-activating substance 134. In this regard, the analysis time may be short, and the diagnostic kit may be prepared with a low-price portable apparatus.

The electrode 120 of the diagnostic kit according to an embodiment of the present invention may consist of a working electrode (WE) 141, a counter electrode (CE) 142, and a reference electrode (RE) 143.

Referring to FIG. 5, the working electrode WE 141 may be in the shape of a square, and the counter electrode CE 142 may be in the shape of a rectangular strip, wherein the counter electrode CE 142 may be provided to wrap the working electrode WE 141 from one side to the other through the lower side of the working electrode WE 141. The reference electrode RE 143 may be in the shape of a square, and may be provided on one side of the working electrode WE 141. Alternatively, the reference electrode RE 143 may be arranged on the upper side of the counter electrode CE 142.

Here, the one side of the working electrode WE 141 may refer to the right side in FIG. 5, and the other side of the working electrode WE 141 may refer to the left side in FIG. 5. In addition, the lower side of the working electrode WE 141 may be a side in a downward direction in FIG. 5, and the upper side of the working electrode WE 141 may be a side in an upper direction in FIG. 5.

An electrode in the art has problems that, due to a small area of a working electrode compared to a counter electrode, an electrode is destroyed or an insulating layer is peeled off during a measurement process. Also, since a counter electrode measures only the right side and lower side of a working electrode, loss of current may occur.

However, regarding the working electrode WE 141, the counter electrode CE 142, and the reference electrode RE 143 according to an embodiment of the present invention, as shown in FIG. 5, the working electrode WE 141 may be a square-shaped electrode, and the counter electrode CE 142 may be provided to wrap the working electrode WE 141 from one side to the other side through the lower side of the working electrode WE 141.

Accordingly, problems that the counter electrode is destroyed, the insulating layer is peeled off, or the current is lost may be solved by providing the counter electrode CE 142 to wrap the left, lower, and right sides of the working electrode WE 141 while increasing the area of the counter electrode CE 142.

Into the plurality of nanowells of the diagnostic kit according to an embodiment of the present invention, particles 150 treated with a fluorescent substance may be inserted. The diagnostic kit according to an embodiment of the present invention may implement an electrochemical analysis method and an optical analysis method at the same time on a single electrode, and for this implementation, the particles 150 treated with a fluorescent substance may be inserted into the plurality of nanowells.

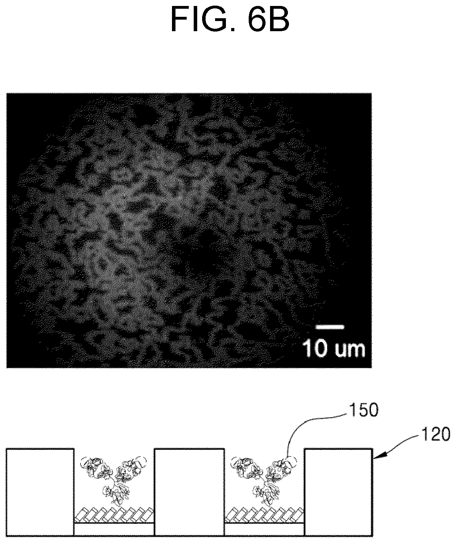

Referring to FIG. 6B, it is preferable that the single particle 150 reacts with the single nanowell. That is, it is preferable that the nanowell and the particle 150 react at a ratio of 1:1.

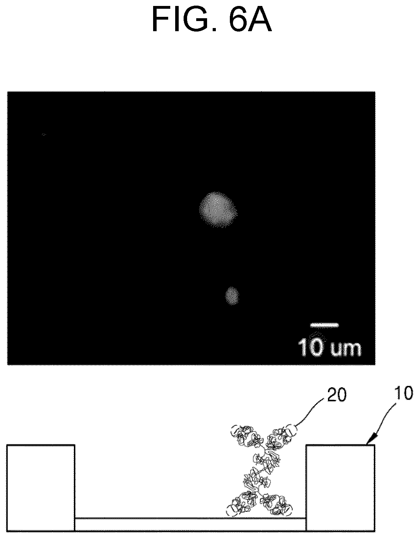

Referring to FIG. 6A, when a particle 20 treated with a fluorescent substance is inserted into an electrode 10 on which a conventional nanowell structure is not formed, problems of lowering the sensitivity due to a protein aggregation reaction may occur. However, the diagnostic kit according to an embodiment of the present invention may have advantages of improving the sensitivity while reducing the protein aggregation reaction by inserting the particles 150 treated with a fluorescent substance while forming the nanowell structure 127.

In particular, in the diagnostic kit according to an embodiment of the present invention, the sensitivity may be improved while reducing the protein aggregation reaction through a 1:1 reaction by inserting the single particle 150 into the single nanowell.

For the 1:1 reaction by inserting the single particle 150 into the single nanowell, the size of the single particle 150 may be preferably larger than 1/2 of the diameter of the single nanowell and smaller than the diameter of the single nanowell.

In detail, the size (diameter) of the single nanowell may be preferably in a range of about 50 nm to about 500 nm, and the size of the single particle 150 may be preferably larger than or equal to 1/2 of the size of the single nanowell and smaller than the size of the single nanowell.

When the size of the single particle 150 is smaller than 1/2 of the size of the single nanowell, two or more particles 150 may be inserted into one nanowell so that the 1:1 reaction may not occur. Thus, the size of single particle 150 may be preferably larger than 1/2 of the size of the single nanowell.

In addition, when the size of the single particle 150 is larger than the size of the single nanowell, the single particle 150 may not be inserted into the single nanowell. Thus, the size of the single particle 150 may be preferably smaller than the size of the single nanowell. As such, by adjusting the size of the single particle 150, the nanowells and the particles 150 may react at a ratio of 1:1.

The nanowell diagnostic kit for diagnosing a cardiovascular disease substance in blood according to an embodiment of the present invention may have the following effects.

The nanowell diagnostic kit for diagnosing a cardiovascular disease substance in blood according to an embodiment of the present invention may have advantages to enabling in vitro diagnosis by performing an electrochemical analysis method through the diagnostic kit on which the nanowell electrode is formed and accordingly by quantifying a very small amount of a target cardiovascular substance in blood.

In addition, the nanowell diagnostic kit for diagnosing a cardiovascular disease substance in blood according to an embodiment of the present invention may have advantages to diagnosing a cardiovascular disease substance in blood quickly and simply by performing an electrochemical analysis using the electron transfer-activating substance 134 together with the electrode 120 on which the nanowell structure 127 is formed.

In the nanowell diagnostic kit for diagnosing a cardiovascular disease substance in blood according to an embodiment of the present invention, the working electrode WE 141 may be formed in the shape of a square, and the counter electrode CE 142 may be provided to wrap the working electrode WE 141 from one side to the lower side through the lower side of the working electrode WE 141.

The nanowell diagnostic kit for diagnosing a cardiovascular disease substance in blood according to an embodiment of the present invention may have an increased area of the counter electrode 142, and include the counter electrode CE 142 to be provided to wrap left, lower, and right sides of the working electrode WE 141, so that the problems that the electrode is destroyed, the insulating layer is peeled off, or the current is lost may be solved.

At the same time, the nanowell diagnostic kit for diagnosing a cardiovascular disease substance in blood according to an embodiment of the present invention may implement both an electrochemical analysis method and an optical method on a single electrode at the same time by reacting the fluorescent substance-treated particles 150 with the nanowells at a ratio of 1:1.

In addition, in the nanowell diagnostic kit for diagnosing a cardiovascular disease substance in blood according to an embodiment of the present invention, the fluorescent substance-treated particles 150 may react with the nanowells at a ratio of 1:1, and thus the sensitivity of the nanowell diagnostic kit may be improved through an optical method by reducing protein aggregation reactions.

As described above, the present invention has been described with reference to one embodiment shown in the drawings, but one of ordinary skill in the art would understood that this is merely exemplary and that various modifications and variations of embodiments are possible therefrom. Therefore, the true technical protection scope of the present invention should be determined by the technical spirit of the appended claims.

## Claims

1. A nanowell diagnostic kit capable of identifying a target substance in blood, the nanowell diagnostic kit comprising:
an electrode on which a plurality of nanowells are formed to accommodate the target substance; and
a marker fixed to each of the plurality of nanowells of the electrode and responding to the target substance, wherein
the target substance is identified through an electrochemical analysis method by applying a current to the electrode.

2. The nanowell diagnostic kit of claim 1, wherein
the target substance is a target cardiovascular substance capable of identifying a cardiovascular disease, and
the marker is a cardiovascular marker that responds to the target cardiovascular substance.

3. The nanowell diagnostic kit of claim 2, wherein
the electrochemical analysis method is performed in a way that,
after the cardiovascular marker responds to the target cardiovascular substance,
a probe binding to the target cardiovascular substance and an electron transfer-activating substance that generates a current through an oxidation-reduction reaction while being bound to the probe are inserted into each of the plurality of nanowells, and
a current value is read by applying a designated voltage to the electrode to identify the target cardiovascular substance.

4. The nanowell diagnostic kit of claim 3, wherein
the electron transfer-activating substance consists of any one of ferrocene, methylene blue, and ferrate.

5. The nanowell diagnostic kit of claim 1, wherein
the electrode on which the plurality of nanowells are formed, consists of a 8-channel electrode, and
different markers are fixed to different channels of the electrode.

6. The nanowell diagnostic kit of claim 1, wherein
the electrode consists of a working electrode, a counter electrode, and a reference electrode,
the working electrode is in the shape of a square,
the counter electrode is in the shape of a rectangular strip, and is provided to wrap the working electrode from one side to the other side via the lower side of the working electrode, and
the reference electrode is in the shape of a square and is provided on one side of the working electrode.

7. The nanowell diagnostic kit of claim 1, wherein
the size of each of the plurality of nanowells is in a range of about 50 nm to about 500 nm.

8. The nanowell diagnostic kit of claim 7, wherein
particles treated with a fluorescent substance are inserted into the plurality of nanowells.

9. The nanowell bio kit for blood cardiovascular diagnosis of claim 8, wherein the size of the particles is greater than 1/2 of the diameter of each of the plurality of nanowells and is smaller than the diameter of each of the plurality of nanowells.

10. The nanowell bio kit for blood cardiovascular diagnosis of claim 9, wherein
one of the particles proceeds a 1:1 reaction when added to one of the plurality of nanowells.
